(19) Europäisches Patentamt
European Patent Office
Office européen des brevets

(11) Publication number: **0 493 602 A1**

(12) **EUROPEAN PATENT APPLICATION published in accordance with Art. 158(3) EPC**

(21) Application number: **91911951.1**

(22) Date of filing: **26.06.91**

(86) International application number: **PCT/JP91/00857**

(87) International publication number: **WO 92/02516 (20.02.92 92/05)**

(51) Int. Cl.5: **C07D 405/04**, C07D 473/34, C07D 473/18, C07D 473/30, C07D 473/06, C07D 473/16, C07D 473/38, C07D 473/32

(30) Priority: **27.07.90 JP 200421/90**
**30.11.90 JP 338649/90**

(43) Date of publication of application: **08.07.92 Bulletin 92/28**

(84) Designated Contracting States: **DE FR GB**

(71) Applicant: **YUKI GOSEI KOGYO CO., LTD.**
**3-24, Hirakawa-cho 2-chome**
**Chiyoda-ku Tokyo 102(JP)**
Applicant: **JAPAN TOBACCO INC.**
**12-16, Higashi Shinagawa 4-chome,**
**Shinagawa-ku, Tokyo 140(JP)**

(72) Inventor: **EBATA, Takashi, Japan Tobacco Inc., Life Science**
**Research Laboratory, 6-2, Umegaoka, Midori-ku**
**Yokohama-shi, Kanagawa-ken 227(JP)**
Inventor: **MATSUSHITA, Hajime, Japan Tobacco Inc., Life**
**Science Research Laboratory, 6-2, Umegaoka, Midori**
**-ku, Yokohama-shi, Kanagawa-ken 227(JP)**
Inventor: **MIZUTANI, Nobuhiro, Yuki Gosei Kogyo Co., Ltd.**
**Tokyo Laboratory, 7-1, Sakashita 3-chome**
**Itabashi-ku, Tokyo 174(JP)**
Inventor: **ITOH, Kazuo, Yuki Gosei Kogyo Co., Ltd.**
**Tokyo Laboratory, 37-1, Sakashita 3-chome**
**Itabashi-ku, Tokyo 174(JP)**

(74) Representative: **Reinhard, Skuhra, Weise**
**Postfach 44 01 51 Friedrichstrasse 31**
**W-8000 München 40(DE)**

(54) **PROCESS FOR PRODUCING 2',3'-DIDEOXY-2',3'-DIDEHYDRONUCLEOSIDE.**

(57) A process for producing 2',3'-dideoxy-2',3'-didehydronucleoside useful as the starting material of medicines readily at a low cost, which comprises the step (a) of reacting a ribonucleoside derivative of general formula (II) with an acid anhydride in the presence of a catalyst to obtain an intermediate of general formula (III) and the step (b) of decarboxylating the intermediate (III) under a neutral or basic condition to obtain 2',3'-dideoxy-2',3'-didehydronucleoside of general formula (I), wherein $R^1$ represents optionally protected hydroxy; $R^2$ and $R^3$ represent each optionally substituted alkyl or phenyl; and B represents a purine or pyrimidine nucleic acid base.

EP 0 493 602 A1

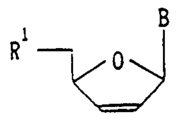

(I)

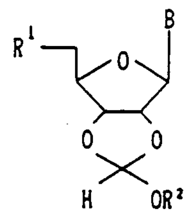

(II)

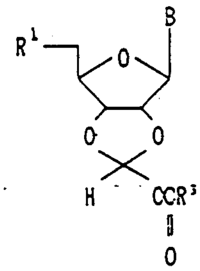

(III)

Technical Field

The present invention relates to a method of manufacturing 2',3'-dideoxy-2',3'-didehydronucleosides.

Background Art

2',3'-dideoxy-2'3'-didehydronucleosides, which are known to exhibit resistances to virus and cancer, known as anti-HIV agents and, thus, useful as raw materials of medicines, are represented by general formula (I) given below:

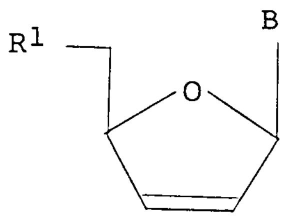

(I)

where $R^1$ denotes a hydroxyl group which may have a protective group in some cases, and B represents purine base or pyrimidine base.

For manufacturing 2',3'-dideoxy-2',3'-didehydronucleosides, known are methods using ribonucleoside as described in, for example, The journal of the Organic Chemistry 39, 30 (1974), The journal of the Organic Chemistry 53, 5170 (1988), and The journal of the Organic Chemistry 54, 2217 (1989), and a method using 2'-deoxynucleoside as described in, for example, Journal of the American Chemical Society 88 1549 (1966), and The journal of the Organic Chemistry 32, 817 (1967).

However, the known methods exemplified above involve many treating steps and use costly reagents as raw materials in many cases. In addition, the known methods are low in yield, unsatisfactory in economy and, thus, are not suitable for the industrial application.

Disclosure of the Invention

The present invention, which has been achieved in an attempt to overcome the above-noted defects inherent in the prior arts, is intended to provide a method which permits manufacturing 2',3'-dideoxy-2',3'-didehydronucleosides easily and at a low cost.

As a result of an extensive research made in an attempt to achieve the object noted above, the present inventors have arrived at the method of the present invention.

According to the present invention, there is provided a method of manufacturing 2',3'-dideoxy-2',3'-didehydronucleosides, comprising the steps of:

(a) carrying out a chemical reaction between a ribonucleoside derivative and an acid anhydride in the presence of a catalyst, said ribonucleoside derivative being represented by general formula (II) given below:

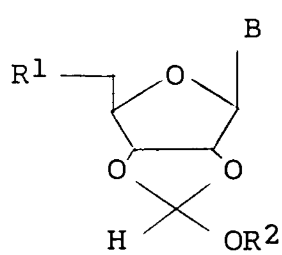

(II)

where $R^1$ is a hydroxyl group which may have a protective group in some cases, $R^2$ is an alkyl or phenyl group which may have substituents in some cases, and B is purine base or pyrimidine base, so as to obtain an intermediate product represented by general formula (III) given below:

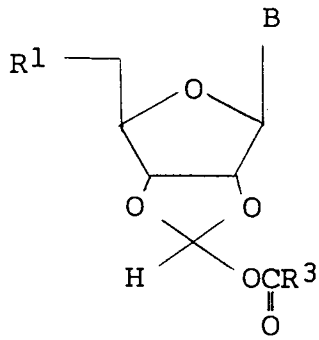

(III)

where $R^1$ is a hydroxyl group which may have a protective group in some cases, $R^3$ is an alkyl or phenyl group which may have substituents in some cases, and B is purine base or pyrimidine base; and (b) subjecting the intermediate product represented by general formula (III), which is obtained in step (a), to a decarboxylation treatment under a neutral or basic condition so as to obtain 2',3'-dideoxy-2',3'-didehydronucleosides represented by general formula (I) given below:

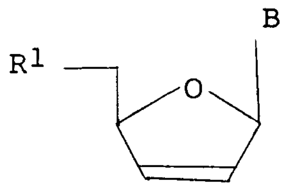

(I)

where $R^1$ is a hydroxyl group which may have a protective group in some cases, and B is purine base or pyrimidine base.

## Brief Description of the Drawings

Fig. 1 is a graph showing changes with time in ratios of the components of the reaction system in Example 2 of the present invention;
Fig. 2 is a graph showing changes with time in ratios of the components of the reaction system in Example 3 of the present invention;
Fig. 3 is a graph showing changes with time in ratios of the components of the reaction system in Comparative Example 1;
Fig. 4 is a graph showing changes with time in ratios of the components of the reaction system in Comparative Example 2;
Figs. 5A to 5C are graphs each showing changes with time in ratios of the components of the reaction system in Comparative Example 3;
Fig. 6 is a graph showing changes with time in ratios of the components of the reaction system in Comparative Example 4;
Fig. 7 is a graph showing changes with time in ratios of the components of the reaction system in Comparative Example 5; and
Figs. 8A and 8B are graphs each showing changes with time in ratios of the components of the reaction system in Comparative Example 6.

## Best Mode of Embodying the Invention

In the method of the present invention, a ribonucleoside derivative represented by general formula (II) is used as a starting material. The ribonucleocide derivative available in the nature can be used in the present invention. Alternatively, the starting material (II) can be obtained by conversion from a ribonucleoside derivative represented by general formula (IV) given below, which can be prepared by a known method;

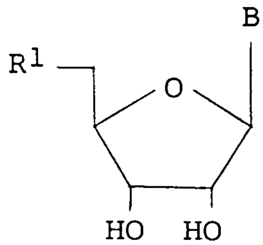

(IV)

where $R^1$ is a hydroxyl group which may have a protective group in some cases, and B is purine base or pyrimidine base.

The conversion from a compound of general formula (IV) into a compound of general formula (II), i.e., the starting material of the present invention, can be performed by the method described in, for example, "Tetrahedron, 23, 2301 (1967)".

$R^1$ included in the ribonucleoside derivative represented by general formula (II) denotes a hydroxyl group which may have a protective group in some cases. The protective group includes, for example, acyl groups such as acetyl, propionyl, pivaloyl and benzoyl; aralkyl groups such as trityl; alkyl carbonyl groups such as ethoxy carbonyl and t-butoxy carbonyl; aryl carbonyl groups such as phenoxy carbonyl; and triorganosilyl groups such as t-butyl dimethyl silyl. Of course, the protective group is not necessarily restricted to those exemplified above. Where the protective group has an additional phenyl group, the phenyl group may be a substituted phenyl group having a substituent of, for example, a halogen atom, an alkyl group, a nitro group or an alkoxy group.

The group $R^2$ included in the rebonucleoside derivative represented by general formula (II) denotes an alkyl group such as methyl or ethyl, or a phenyl group which may have a substituent in some cases.

Further, the group B included in the ribonucleoside derivative represented by general formula (II) denotes a series of bases consisting of pyrimidine bases and purine bases. The pyrimidine base includes, for example, uracil, cytosine, thymine, 5-fluorouracil, 5-chlorouracil, 5-bromouracil, 5-iodouracil, 5-ethyluracil, 5-trifluoromethyl uracil, and 5-carboxyl uracil. On the other hand, the purine base includes, for example, adenine, guanine, hypoxanthine, xanthine, 2-chloropurine, 6-chloropurine, 2,6-dichloropurine, 2-amino-6-chloropurine, 2,6-diaminopurine, 6-mercaptopurine, 6-methyl thiopurine and 2-aminopurine. Of course, the pyrimidine base and the purine base need not be restricted to those exemplified above.

The method of the present invention comprises step (a) in which a chemical reaction is carried out between a ribonucleoside derivative (II) and an acid anhydride so as to obtain an intermediate product (III) and step (b) in which the intermediate product (III) is subjected to decarboxylation treatment by heating or the like so as to obtain a desired product of 2',3'-dideoxy-2',3'-didehydronucleoside. The reaction in step (a) is carried out in the presence of a catalyst. On the other hand, a free acid is neutralized by, for example, the addition of a base in step (b) so as to carry out the decarboxylation under a neutral or basic condition. By these steps (a) and (b), the desired product of 2',3'-dideoxy2',3'-didehydronucleosides (I) can be obtained with a high yield.

In each of steps (a) and (b), the reaction should be carried out at 0 to 200°C for 30 minutes to 24 hours.

The acid anhydride reacting with the ribonucleoside derivative (II) in step (a) includes, for example, acetic anhydride, chloroacetic anhydride, dichloroacetic anhydride, trichloroacetic anhydride, trifluoroacetic anhydride, benzoic anhydride, and propionic anhydride. These anhydrides can be used singly or together with another solvent, as desired, such as xylene, N,N-dimethyl formamide, butyl acetate or nitrobenzene.

The group $R^3$ included in the intermediate product (III) represents an acyl group portion of the acid anhydride noted above.

The catalyst used in step (a) includes, for example, solid acids such as hydrous zirconium oxide, hydrous titanium oxide, hydrous aluminum oxide, hydrous tin oxide, titanium oxide, zirconium oxide, silica gel, alumina, and zeolite; organic acids such as acetic acid, propionic acid, and p-toluene-snlfonic acid; mineral acids such as hydrochloric acid, sulfuric acid and phosphoric acid; and an acidic ion exchange resin. It is particularly desirable to use hydrous zirconium oxide (HZO) as the catalyst. In this case, the intermediate product (III) can be accumulated within the reaction system in a short time.

The reaction in step (a) proceeds even in the absence of a catalyst. In this case, however, the forming rate of the intermediate product (III) is very low as in the conventional method. The intermediate product (III) formed in step (a) is partially converted into the desired product of 2',3'-dideoxy-2', 3'-didehydronucleoside (I) in step (a). However, the glycoside bond of compound (I) thus formed is cleaved by the acid present

within the reaction system and, thus, compound (I) is decomposed. It follows that it is desirable to finish the reaction for forming the intermediate product (III) in a short time. In other words, the yield of the desired product (I) is lowered with increase in the reaction time.

The intermediate product (III) formed in step (a) is subjected to a decarboxylation treatment so as to convert the intermediate product (III) into the desired compound of 2',3'-dideoxy-2',3'-didehydronucleoside (I). The decarboxylation treatment in step (b) is carried out in a neutral or basic reaction system and, thus, a base is added to the reaction system in step (b). If the decarboxylation treatment is carried out under an acidic condition, the glycoside bond of the nucleoside is cleaved, with the result that the nucleoside is decomposed into a base and a sugar part. In other words, the expression "neutral or basic" condition referred to above denotes that the reaction system is not acidic. However, the present invention does not exclude the case where the reaction system is temporarily rendered acidic. The base used in step (b) for rendering the reaction system neutral or basic includes, for example, amines such as triethyl amine, n-tributyl amine, and pyridine; metal alkoxides such as sodium methoxide and sodium ethoxide; basic ion exchange resin; and inorganic bases such as sodium hydrogencarbonate and sodium hydride. Under such a neutral or basic condition, the ribonucleoside derivative (II) does not react with the acid anhydride so as to stop formation of the intermediate product (III).

After the reaction, the reaction product is purified by the ordinary method such as the extraction method or recrystallization method so as to obtain the desired compound of 2',3'-dideoxy-2',3'-didehydronucleosides represented by general formula (I). In some cases, a de-protection treatment is applied for the purifying purpose, as desired.

As described above, the method of the present invention comprises step (a) in which a chemical reaction between a ribonucleoside derivative (II) and an acid anhydride is carried out in the presence of a catalyst so as to obtain an intermediate product (III) with a high yield, and step (b) in which the intermediate product (III) is subjected to a decarboxylation treatment by heating. What should be noted is that, if step (b) is carried out under an acidic condition, the glycoside bond of the intermediate product (III) is cleaved, with the result that the purine base or pyrimidine base denoted by B in general formula (III) is separated. In the method of the present invention, however, the reaction system in step (b) is rendered neutral or basic so as to prevent the intermediate product (III) from being decomposed by an acid and, thus, to obtain the desired compounds of 2',3'-dideoxy-2',3'-didehydronucleosides with a high yield.

Example 1

Manufacture of 2',3'-dideoxy-2',3'-didehydrothymidine:

0.02 g (0.11 mmol) of hydrated p-toluene sulfonate was added to a suspension prepared by adding 2.00 g (7.75 mmol) of 5-methyl uridine to 10 mℓ of methyl orthoformate. The resultant mixture was kept stirred overnight at room temperature. Then, the liquid material was neutralized with 21 μℓ of 28% sodium methoxide in methanol, followed by removing the solvent under a reduced pressure. Then, 12 mℓ of acetic anhydride was added to the residue, and the mixture was heated at 100 °C for one hour.

In the next step, 1.50 g of hydrous zirconium oxide was added to the reaction system, and the system was heated at 130 °C for 3 hours, followed by adding 6 mℓ of n-tributyl amine to the system so as to carry out the reaction at 130 °C for 4 hours. After the reaction, the reaction mixture was cooled to room temperature and poured into an aqueous solution of sodium carbonate. Then, the reaction product was extracted three times with chloroform. The extracted liquid material was condensed under reduced pressure, and 30 mℓ of 25% ammonia water was added to the residue, which was kept stirred overnight under room temperature. The resultant liquid material was washed with hexane and, then, the solvent was removed under a reduced pressure. Finally, the residue thus obtained was purified by using a slica gel column chromatography (chloroform : methanol = 20 : 1) so as to obtain a desired compound of 2',3'-dideoxy-2',3'-didehydrothymidine in an amount of 1.50 g (6.69 millimols). The product yield was 86%. The melting point of the product was 164-166 °C (decomposition).

Example 2

1.5 ml of acetic anhydride was added to 0.5 mmol of 5'-O-acetyl-2',3'-O-methoxymethylidene uridine used as a starting material, and the mixture was heated at 100 °C for one hour. Then, 0.10 g of hydrous zirconium oxide was added to the mixture, and the mixture was heated to 130 °C, followed by adding 1.5 mℓ of triethylamine to the mixture one hour later and further heating the reaction system. Measured were changes with time in the percentages of the starting material, intermediate product, formed product, and

decomposition product in the reaction system, starting with the time of adding hydrous zirconium oxide. Fig. 1 is a graph showing the results. In the graph of Fig. 1, the ordinate denotes the percentage of the materials present in the reaction system, with the abscissa representing the time starting with the hydrous zirconium oxide addition. The symbols used in Fig. 1 represents the materials present in the reaction system, which are defined as follows:

- ● - : starting material

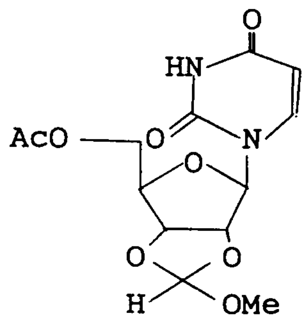

- X - : intermediate product

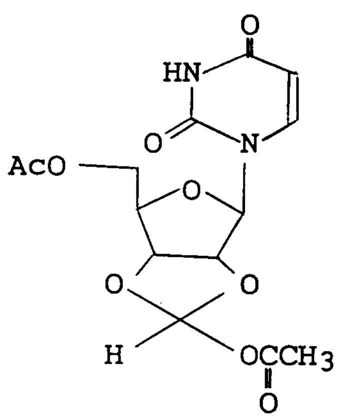

- O - : formed product

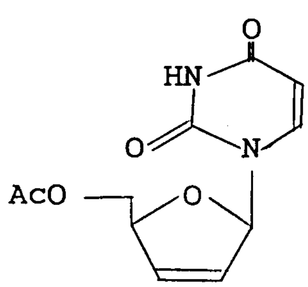

- □ - : Acetylated formed product

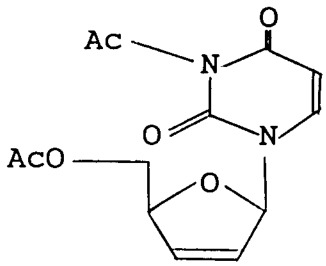

- △ - : Decomposition product

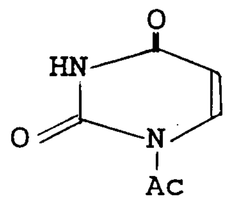

The symbols given above are similarly used in Figs. 2 to 8 which are referred to later.

Example 3

Changes with time in the percentage of the materials present in the reaction system were measured as in Example 2, except that 1.5 ml of triethylamine was added every hour after the addition of the hydrous zirconium oxide in Example 3. Fig. 2 shows the results.

As apparent from Figs. 1 and 2, the decomposition product was detected in only three hours later in Example 2 in which the amine was added only once. On the other hand, the decomposition product was not detected more than 5 hours in Example 3 in which the amine was added every hour. It should be noted in this connection that the amine added to the reaction system is consumed for the neutralization of acetic acid which is generated in the reaction system to form the intermediate product, with the result that it is difficult to maintain the reaction system neutral or basic.

Comparative Example 1
(No amine addition)

Changes with time in the percentage of the materials present in the reaction system were measured as in Example 2, except that triethylamine was not added in Comparative Example 1. Fig. 3 shows the results.

Comparative Example 2
(No amine addition)

Changes with time in the percentage of the materials present in the reaction system were measured as in Comparative Example 1, except that acetic anhydride was used in an amount of 4.0 mℓ and hydrous zirconium oxide was used in an amount of 0.50 g in Comparative Example 2. Fig. 4 shows the results.

Comparative Example 3
(No amine addition)

Changes with time in the percentage of the materials present in the reaction system were measured as in Comparative Example 1, except that acetic anhydride was used in an amount of 4.0 mℓ and the reaction temperature was set at three different levels, i.e., 120 ° C, 130 ° C and 140 ° C in Comparative Example 3. Figs. 5A to 5C show the results.

Comparative Example 4
(No amine addition and Use of Silicon Dioxide)

Changes with time in the percentage of the materials present in the reaction system were measured as in Comparative Example 1, except that acetic anhydride was used in an amount of 4.0 mℓ and silicon dioxide ($SiO_2$) was used as a catalyst in Comparative Example 4. Fig. 6 shows the results.

Comparative Example 5
(No amine addition)

Changes with time in the percentage of the materials present in the reaction system were measured as in Example 2, except that triethylamine was not added and 3 equivalents of acetic acid was used as a catalyst in Comparative Example 5. Fig. 7 shows the results.

Comparative Example 6
(No addition of amine and catalyst)

Changes with time in the percentage of the materials present in the reaction system were measured as in Comparative Example 5, except that acetic anhydride was used in an amount of 4.0 mℓ and acetic acid as a catalyst was not used, and the reaction temperature was set at two different levels, i.e., 120°C and 130°C. Figs. 8A and 8B show the results.

As shown in Figs. 8A and 8B, the rate of decrease in the amount of the starting material is low in the case where a catalyst is not used. Clearly, the conversion rate from the starting material into the intermediate product is low. In the case of using hydrous zirconium oxide as a catalyst, however, the amount of the starting material is rapidly decreased to form the intermediate product, as apparent from Figs. 1 to 5C. In the case of using silicon dioxide (Fig. 6) and acetic acid as a catalyst (Fig. 7), the rate of decrease in the amount of the starting material is somewhat improved. However, the improvement is insufficient.

Even if hydrous zirconium oxide is used as a catalyst, it is difficult to obtain a satisfactory effect of the present invention if an amine is not added to the reaction system, as seen from Figs. 3 to 5C. To be more specific, if an amine is not added, the decomposition product is detected shortly after initiation of the reaction. In addition, the percentage of the desired product becomes constant or is lowered in a short time, and the percentage of the decomposition product is rapidly increased. Where an amine is added to the reaction system, however, the decomposition product is not detected over a relatively long time after initiation of the reaction, as shown in Figs. 1 and 2. Also, the formation of the desired product is kept increased over a long time, and the percentage of the decomposition product is increased very moderately.

Example 4

Manufacture of 5'-0-acetyl-2',3'-dideoxy-2',3'-didehydroinosine

3.0 mℓ of acetic anhydride was added to 0.9 mmol of a starting material of 5'-O-acetyl-2',3'-O-methoxy methylidene inosine, and the mixture was heated at 100°C for one hour. Then, 200 mg of hydrous zirconium oxide (HZO) was added to the heated mixture, and the mixture was further heated to 130°C. Further, 3.0 mℓ of n-tributylamine was added 0.5 hour later, and the heating was further continued so as to obtain 5'-O-acetyl-2',3'-dideoxy-inosine. The yield of 5'-O-acetyl-2',3'dideoxy- 2',3'-didehydroinosine at 0.5 hour after the addition of the n-tributyl amine was 19%.

Examples 5-7

5'-0-acetyl-2',3'-dideoxy- 2',3'-didehydro inosine was manufactured in the same treatment as in Example 4, except that the base shown in Table 1 was used in these Examples in place of the catalyst used in Example 4 and that the reaction time between the addition of the catalyst and the addition of n-tributylamine was changed as shown in Table 1 in Examples 5-7. Table 1 also shows the product yield. It should be noted that "HTO" and "SK1B" shown in Table 1 represent hydrous titanium oxide and an acidic ion exchange resin available on the market, respectively. Further, in Example 6, $SiO_2$ was used in an amount three times as large as in the ordinary case.

Table 1

| Example | Catalyst (mg) | Reaction Time (hour) | Yield (%) |
|---|---|---|---|
| 4 | HZO 200 | 0.5 | 19 |
| 5 | HTO 200 | 0.75 | 17 |
| 6 | $SiO_2$ 600 | 1.0 | 4 |
| 7 | SK1B 200 | 3.0 | 4 |

Examples 8 and 9

5'-0-acetyl-2',3'-dideoxy- 2',3'-didehydroinosine was manufactured in the same treatment as in Example 4, except that, in Example 8, 2,6-lutidine was used as a base added to the reaction system and the reaction time between the HZO addition and the base addition was 1.0 hour, and that, in Example 9, 2-dimethylamino pyridine (2-DMAP) was used as a base added to the reaction system. The product yield was 4% in each of Examples 8 and 9.

Examples 10 to 13

5'-O-acetyl-2',3'-dideoxy- 2',3'-didehydroinosine was manufactured in the same treatment as in Example 4, except that the reaction temperature and reaction time ($T_1$) during the period between the HZO addition and the n-tributylamine addition, and the time ($T_2$) between the n-tributylamine addition and the yield measurement were set as shown in Table 2. Table 2 also shows the product yield. Incidentally, the temperature was elevated to 130 ° C in Example 10 during one hour after the amine addition.

Table 2

| Example | Reaction Temp. | $T_1$ (hour) | $T_2$ (hour) | Yield (%) |
|---|---|---|---|---|
| 10 | 100 ° C | 4.0 | 1.5 | 15 |
| 11 | 110 ° C | 2.0 | 1.0 | 16 |
| 12 | 120 ° C | 1.0 | 1.0 | 16 |
| 13 | 140 ° C | 0.5 | 0.25 | 9 |

Examples 14 to 17

5'-O-acetyl-2',3'-dideoxy- 2',3'-didehydroinosine was manufactured in the same treatment as in Example 4, except that a mixed liquid consisting of acetic anhydride ($Ac_2O$) and nitrobenzene ($PhNO_2$) in mixing amounts shown in Table 3 was used as a solvent in these Examples 14 to 17 in place of acetic anhydride used in Example 4. In Example 14, however, the reaction time between the HZO addition and the n-tributylamine addition was set at 0.75 hour. Also, in Example 17, 1.0 mℓ (4.5 mmol) of n-tributylamine was added to the reaction mixture. Table 3 also shows the product yield in each of these Examples.

Table 3

| Example | $Ac_2O$ (mmol) | $PhNO_2$ (mℓ) | Yield (%) |
|---|---|---|---|
| 14 | 2.25 | 2.8 | 7 |
| 15 | 4.50 | 2.6 | 21 |
| 16 | 9.0 | 2.2 | 18 |
| 17 | 4.5 | 2.6 | 9 |

Example 18

5'-O-acetyl-2',3'-dideoxy- 2',3'-didehydroinosine was manufactured in the same treatment as in Example 4, except that 3.0 mℓ of propionic anhydride was used as solvent in Example 18 in place of acetic anhydride used in Example 4. The product yied in Example 18 was 21%.

Comparative Example 7

5'-0-acetyl-2',3'-dideoxy- 2',3'-didehydroinosine was manufactured in the same treatment as in Example 4, except that a solvent was not used and that n-tributylamine was added to the reaction system at 5 hours after initiation of the reaction in Comparative Example 7. The product yied in Comparative Example 7 was 0.5%.

Example 19

3.0 mℓ of acetic anhydride was added to 0.9 mmol of the starting material of 5'-O-dimethoxymethyl-2',3'-methoxymethylidene inosine, and the mixture was heated at 100°C for one hour. Then, 400 mg of HZO was added to the mixed solution, and the solution was heated at 130°C. Further, 200 mg of HZO was added to the reaction system one hour later. In the next step, 3.0 mℓ of n-tributylamine was added to the reaction system 1.5 hours after the initial HZO addition, and the reaction system was further heated so as to obtain 5'-O-acetyl-2',3'-dideoxy- 2',3'-didehydro inosine. The yield of 5'-O-acetyl-2',3'-dideoxy-2',3'-didehydroino-sine 0.5 hour after the addition of n-tributylamine was 16%.

Example 20

3.0 mℓ of acetic anhydride was added to 0.9 mmol of the starting material of 5'-O-acetyl-2',3'-ethoxy methylidene inosine, and the resultant mixture was heated at 100°C for one hour. Then, 200 mg of HZO was added to the mixed solution, and the solution was heated at 130°C. In the next step, 3.0 mℓ of n-tributylamine was added to the reaction system 0.5 hour after the HZO addition, and the reaction system was further heated so as to obtain 5'-O-acetyl-2',3'-dideoxy-2',3'-didehydroinosine. The yield of 5'-0-acetyl2',3'-dideoxy-2',3'-didehydroinosine 0.5 hour after the addition of n-tributylamine was 21%.

Example 21

Manufacture of 5'-O-(p-chlorobenzoyl)-2',3'-dideoxy-2',3'-didehydroinosine

1.5 mℓ of acetic anhydride was added to 0.45 mmol of the starting material of 5'-O-(p-chlorobenzoyl)-2',3'-methoxymethylideneinosine, and the resultant mixture was heated at 100°C for one hour. Then, 100 mg of HZO was added to the mixed solution, and the solution was heated at 130°C. In the next step, 1.5 mℓ of n-tributylamine was added to the reaction system 1.25 hour after the HZO addition, and the reaction system was further heated so as to obtain 5'-O-(p-chlorobenzoyl)-2',3'-dideoxy-2',3'-didehydroinosine. The yield of 5'-O-(p-chlorobenzoyl) -2',3'-dideoxy-2',3'-didehydro inosine 0.25 hour after the addition of n-tributylamine was 11%.

Example 22

5'-O-(p-chlorobenzoyl)-2',3'-dideoxy-2',3'-didehydroinosine was manufactured with the treatment equal to that of Example 21, except that the catalyst used and the reaction time between the catalyst addition and the n-tributylamine addition were changed as shown in Table 4. Table 4 also shows the product yield. The abbreviations "HZO" and "HTO" shown in Table 4 are equal to those indicated previously.

Table 4

| Example | Catalyst (mg) | Reaction Time | Yield |
|---|---|---|---|
| 21 | HZO 100 | 1.25 hour | 11% |
| 22 | HTO 100 | 1.25 hour | 13% |

Comparative Example 8

5'-0-(p-chlorobenzoyl)-2',3'-dideoxy- 2',3'-didehydroinosine was manufactured with the treatment equal to that of Example 21, except that the catalyst was not used and that n-tributylamine was added to the reaction system 5 hours after the initiation of the reaction in Comparative Example 8. The product yield in Comparative Example 8 was 0.7%.

Example 23

Manufacture of 5'-O-(t-butyl diphenyl silyl)-2',3'-dideoxy-2',3'-didehydroinosine

1.5 mℓ of acetic anhydride was added to 0.45 mmol of the starting material of 5'-O-(t-butyl diphenyl silyl)-2',3'-O-methoxymethylidene inosine, and the resultant mixture was heated at 100 °C for one hour. Then, 100 mg of HZO was added to the mixed solution, and the solution was heated at 130 °C. In the next step, 1.5 mℓ of n-tributylamine was added to the reaction system 0.75 hour after the HZO addition, and the reaction system was further heated so as to obtain 5'-O-(t-butyl diphenyl silyl)-2',3'-dideoxy-2',3'-didehydroinosine. The yield of 5'-O-(t-butyl diphenyl silyl)-2',3'- dideoxy-2',3'-didehydroinosine 0.5 hour after the addition of n-tributylamine was 17%.

Examples 24 to 32

The treatments similar to those of Example 23 were carried out so as to obtain 5'-O-(t-butyl diphenyl silyl)-2',3'-dideoxy-2',3'-didehydroinosine, except that the catalysts shown in Table 5 were used in these Examples 24 to 32 in place of HZO used in Example 23 and that the reaction time between the catalyst addition and the addition of n-tributyl amine was changed in Examples 24 to 32 as shown in Table 5. Table 5 also shows the product yield in each of these Examples 24 to 32. It should be noted that in Example 27 $SiO_2$ was used as a catalyst in an amount three times as large as in the ordinary case. Also, $TiO_2$ used in Example 28 was of rutil type. Further, the abbreviations "HAO" and "HSnO" shown in Table 5 denote hydrous aluminum oxide and hydrous tin oxide, respectively. Likewise, "AcOH" and "MS4A" in Table 5 denote acetic acid and molecular shieve 4A (i.e., zeolite available on the market), respectively.

Table 5

| Example | Catalyst (mg) | Recaction Time | Yield (%) |
|---|---|---|---|
| 23 | HZO 100 | 0.75 hour | 17 |
| 24 | HTO 100 | 0.75 hour | 14 |
| 25 | HAO 100 | 5.0 hours | 3 |
| 26 | HSnO 100 | 5.0 hours | 5 |
| 27 | $SiO_2$ 300 | 2.5 hours | 4 |
| 28 | $TiO_2$ 100 | 5.0 hours | 6 |
| 29 | $ZrO_2$ 100 | 2.0 hours | 3 |
| 30 | $A\ell_2O_3$ 300 | 5.0 hours | 4 |
| 31 | AcOH 81 | 5.0 hours | 7 |
| 32 | MS4A 100 | 2.5 hours | 3 |

Examples 33 to 35

The treatments similar to those of Example 23 were carried out so as to obtain 5'-O-(t-butyl diphenyl silyl)-2',3'-dideoxy-2',3'-didehydroinosine, except that a mixed solution containing acetic anhydride ($Ac_2O$) and xylene in amounts as shown in Table 6 was used as a solvent in place of acetic anhydride used in Example 23, and that the reaction time between the HZO addition and the n-tributylamine addition was set at one hour in Examples 33 to 35.

Table 6

| Example | $Ac_2O$ (mmol) | Xylene (m$\ell$) | Yield (%) |
|---|---|---|---|
| 33 | 1.125 | 1.4 | 20 |
| 34 | 2.25 | 1.3 | 16 |
| 35 | 4.5 | 1.1 | 16 |

Example 36

The treatments similar to those of Example 23 were carried out so as to obtain 5'-O-(t-butyl diphenyl silyl)-2',3'-dideoxy-2',3'-didehydroinosine, except that a mixed solution containing 2.25 mmol of acetic anhydride and 1.3 m$\ell$ of xylene was used as a solvent in place of acetic anhydride used in Example 23, an acidic ion exchange resin was used as a catalyst in Example 36, and that the reaction time between the catalyst addition and the n-tributylamine addition was set at 3 hours in Example 36. The product yield in Example 36 was 21%.

Examples 37 to 39

The treatments similar to those of Example 23 were carried out so as to obtain 5'-O-(t-butyl diphenyl silyl)-2',3'-dideoxy-2',3'-didehydroinosine, except that a mixed solution containing 2.25 mmol of acetic anhydride and 1.3 m$\ell$ of xylene was used as a solvent in place of acetic anhydride used in Example 23, the reaction time between the HZO addition and the subsequent addition of a base was set at 0.5 hour in Examples 37 to 39, and that the base materials shown in Table 7 were used in Examples 37 to 39 in place of n-tributylamine used in Example 23. Table 7 also shows the product yield.

Table 7

| Example | Base (mℓ) | Yield (%) |
| --- | --- | --- |
| 37 | 2,6-ruthidine 1.5 | 15 |
| 38 | di-isobutylamine 1.5 | 4 |
| 39 | dicyclohexylamine 1.5 | 8 |

Example 40 to 44

The treatments similar to those of Example 23 were carried out so as to obtain 5'-O-(t-butyl diphenyl silyl)-2',3'-dideoxy-2',3'-didehydroinosine, except that a mixed solution containing 2.25 mmol of acetic anhydride and 1.3 mℓ of xylene was used as a solvent in place of acetic anhydride used in Example 23, the reaction time between the HZO addition and the subsequent addition of a base was set as shown in Table 8, and that the reaction temperature during the reaction time noted above was set as shown in Table 8. In Example 44, n-tributylamine was added to the reaction system in an amount of 0.5 mℓ (2.25 mmol). Table 8 also shows the product yield.

Table 8

| Example | Reaction Temp. | Reaction Time | Yield (%) |
| --- | --- | --- | --- |
| 40 | 100°C | 1.5 hours | 24 |
| 41 | 110°C | 1.0 hour | 15 |
| 42 | 120°C | 1.0 hour | 28 |
| 43 | 140°C | 0.5 hour | 8 |
| 44 | 130°C | 0.5 hour | 22 |

Comparative Example 9

The treatments similar to those of Example 23 were carried out, except that a catalyst was not used and that n-tributylamine was added to the reaction system in an amount of 3.0 mℓ in Comparative Example 9. The amount of 5'-O-(t-butyl diphenyl silyl)-2',3'-dideoxy-2',3'-didehydroinosine was measured 6.0 hours after the initiation of the reaction. The product yield was found to be 1%.

Comparative Example 10

The treatments similar to those of Example 23 were carried out, except that a catalyst was not used and a mixture liquid consisting of 2.25 mmol of acetic anhydride and 1.3 mℓ of xylene was used as a solvent in place of acetic anhydride used in Example 23, and that n-tributyl-amine was added to the reaction system in an amount of 3.0 mℓ in Comparative Example 10. The amount of 5'-O-(t-butyl diphenyl silyl)-2',3'-dideoxy- 2',3'-didehydroinosine was measured 5.0 hours after the initiation of the reaction. The product yield was found to be 1%.

Example 45

Manufacture of $N^6$-5'-O-dibenzyl-2',3'-dideoxy-2',3'-didehydroadenosine

490 mg (1.0 mmol) of $N^6$-5'-O-dibenzyl-2',3'-methoxymethylidene adenosine was dissolved in 1.0 mℓ of acetic anhydride. Then, 200 mg of hydrous zirconium oxide was added to the resultant solution, followed by heating the mixture at 100°C for 3.5 hours. Then, 1.0 mℓ of n-tributylamine was added to the heated mixture so as to provide a reaction system. Further, the reaction system was heated at 130°C for 3.5 hours

so as to obtain a desired product of $N^6$-5'-0-dibenzyl-2',3'-dideoxy-2', 3-didehdroadenosine. The product yield measured by a high performance liquid chromatography (HPLC) was found to be 58%.
$^1$H-NMR (δ ppm, $CDCl_3$):
8.42 (1H, s, base), 7.96 (1H, s, base), 7.35-7.20 (10H, m, Ph), 7.15 (1H, m, 1'-H), 6.36 (1H, ddd, J = 1.6Hz, 1.7Hz, 6.0Hz, 2'-H), 6.02 (1H, ddd, J = 1.5Hz, 2.2Hz, 6.0Hz, 3'-H), 5.5-4.9 (3H, bs, N-$CH_2$ and NH), 5.05 (1H, m, 4'-H), 4.47 (2H, m, 5'-H), 3.61 (2H, d, J = 3.7Hz, 0-$CH_2$).

Comparative Example 11

390 mg (1.0 mmol) of $N^6$-5'-0-diacetyl-2',3'-methoxymethylidene adenosine was dissolved in 1.0 mℓ of acetic anhydride. Then, the treatments as in Example 45 were carried out, resulting in failure to obtain of $N^6$-5'-0-dibenzyl-2',3'-dideoxy-2',3'-didehydroadenocine.

Example 46

Manufacture of 5'-O-benzyl-2',3'-dideoxy-2',3'-didehydroinosine

400 mg (1.0 mmol) of 5'-O-benzyl-2',3'-methoxymethylidene inosine was dissolved in 1.0 mℓ of acetic anhydride. Then, the treatments as in Example 45 were carried out. As the result, obtained was the desired product of 5'-O-benzyl-2',3'-dideoxy-2',3'-didehydroinosine. The product yield was 48%.
$^1$H-NMR (δ ppm, $CDCl_3$):
8.06 (1H, s, base), 7.91 (1H, s, base), 7.36-7.30 (5H, m, Ph), 6.99 (1H, m, 1'-H), 6.40 (1H, m, 2'-H), 6.12 (1H, m, 3'-H), 5.27 (2H, s, $CH_2$), 5.16 (1H, m, 4'-H), 4.30 (1H, dd, J = 3.6Hz, 12.4 Hz, 5'-Ha), 4.23 (1H, dd, J = 3.2Hz, 12.4 Hz, 5'-Hb)

As described above in detail, the method of the present invention makes it possible to manufacture easily and at a low cost 2',3'-dideoxy-2',3'-didehydronucleosides without using costly reagents and chemicals which involve dangers in the handling.

**Claims**

**1.** A method of manufacturing 2',3'-dideoxy-2',3'-didehydronucleosides, comprising the steps of:

a) carrying out a chemical reaction between a ribonucleoside derivative and an acid anhydride in the presence of a catalyst, said ribonucleoside derivative being represented by general formula (II) given below:

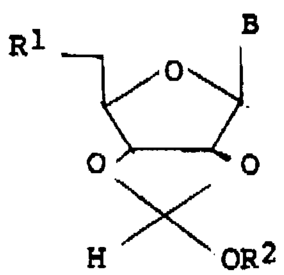

(II)

where $R^1$ is a hydroxyl group which may have a protective group in some cases, $R^2$ is an alkyl or phenyl group which may have a substituent in some cases, and B is purine base or pyrimidine base, so as to obtain an intermediate product represented by general formula (III) given below:

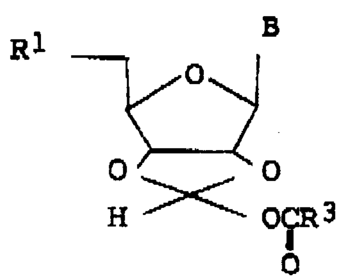

(III)

where $R^1$ is a hydroxyl group which may have a protective group in some cases, $R^3$ is an alkyl or phenyl group which may have a substituent in some cases, and B is purine base or pyrimidine base; and

b) subjecting the intermediate product represented by general formula (III), which is obtained in step a), to a decarboxylation treatment under a neutral or basic condition so as to obtain 2',3'-dideoxy-2',3'-didehydronucleosides represented by general formula (I) given below:

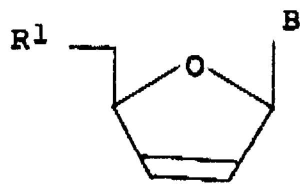

(I)

where $R^1$ is a hydroxyl group which may have a protective group in some cases, and B is purine base or pyrimidine base.

2. The method of manufacturing 2',3'-dideoxy-2',3'-didehydronucleosides according to claim 1, wherein said catalyst is selected from the group consisting of a solid acid catalyst, an organic acid, a mineral acid and an acidic ion exchange resin.

3. The method of manufacturing 2',3'-dideoxy-2',3'-didehydronucleosides according to claim 1, wherein said step b) is carried out in the presence of at least one base selected from the group consisting of amines, alkoxides, basic ion exchange resin and inorganic bases.

**Amended claims**

1. A method of manufacturing 2',3'-dideoxy-2',3'-didehydronucleosides, comprising the steps of:

a) carrying out a chemical reaction between a ribonucleoside derivative and an acid anhydride in the presence of a catalyst, said ribonucleoside derivative being represented by general formula (II) given below:

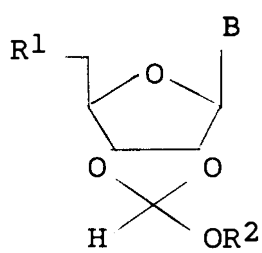

(II)

where $R^1$ is a hydroxyl group which may have a protective group in some cases, $R^2$ is an alkyl or phenyl group which may have a substituent in some cases, and B is purine base or pyrimidine base, so as to obtain an intermediate product represented by general formula (III) given below:

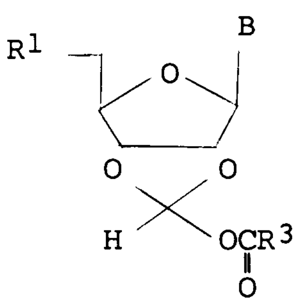

(III)

where $R^1$ is a hydroxyl group which may have a protective group in some cases, $R^3$ is an alkyl or phenyl group which may have a substituent in some cases, and B is purine base or pyrimidine base; and

b) subjecting the intermediate product represented by general formula (III), which is obtained in step a), to a decarboxylation treatment under a neutral or basic condition so as to obtain 2',3'-dideoxy-2',3'-didehydronucleosides represented by general formula (I) given below:

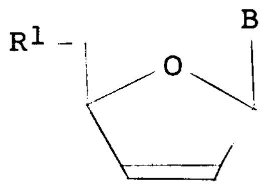

(I)

where $R^1$ is a hydroxyl group which may have a protective group in some cases, and B is purine base or pyrimidine base.

**2.** The method of manufacturing 2',3'-dideoxy-2',3'-didehydronucleosides according to claim 1, wherein said catalyst is selected from the group consisting of a solid acid catalyst, an organic acid, a mineral acid and an acidic ion exchange resin.

**3.** The method of manufacturing 2',3'-dideoxy-2',3'-didehydronucleosides according to claim 1, wherein said step b) is carried out in the presence of at least one base selected from the group consisting of amines, alkoxides, basic ion exchange resin and inorganic bases.

**4.** (addition). The method of manufacturing 2', 3'-dideoxy-2',3'-didehydronucleosides according to claim 1, wherein said group B included in general formulas (I) to (III) is an uracil derivative.

**5.** (addition). The method of manufacturing 2', 3'-dideoxy-2',3'-didehydronucleosides according to claim 1, wherein said group B included in general formulas (I) to (III) is a hypoxanthin derivative.

**6.** (addition). The method of manufacturing 2', 3'-dideoxy2',3'-didehydronucleosides according to claim 1, wherein said group B included in general formulas (I) to (III) is an adenine derivative.

**7.** (addition). The method of manufacturing 2', 3'-dideoxy-2',3'-didehydronucleosides according to claim 3, wherein said group B included in general formulas (I) to (III) is an uracil derivative.

**8.** (addition). The method of manufacturing 2', 3'-dideoxy-2',3'-didehydronucleosides according to claim 3, wherein said group B included in general formulas (I) to (III) is a hypoxanthin derivative.

**9.** (addition). The method of manufacturing 2', 3'-dideoxy2',3'-didehydronucleosides according to claim 3, wherein said group B included in general formulas (I) to (III) is an adenine derivative.

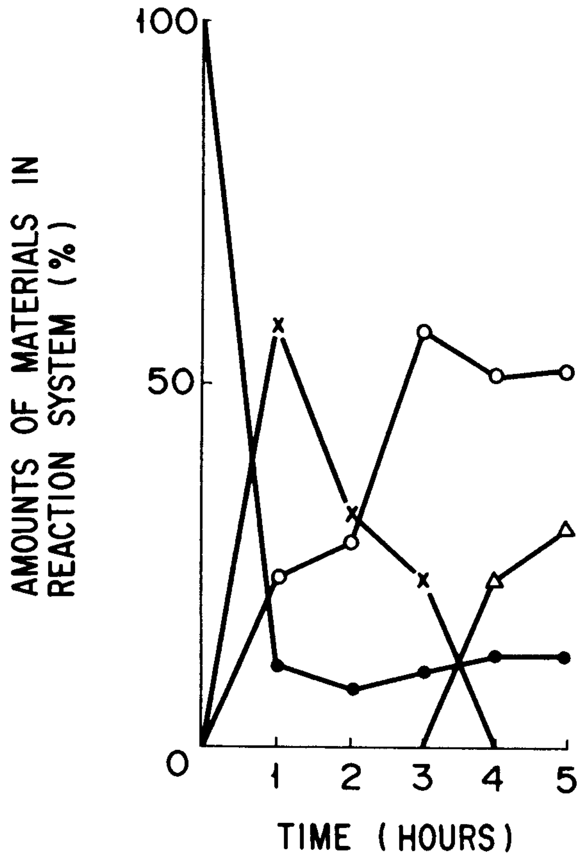
100
50
0
AMOUNTS OF MATERIALS IN REACTION SYSTEM (%)
1 2 3 4 5
TIME (HOURS)

FIG. 1

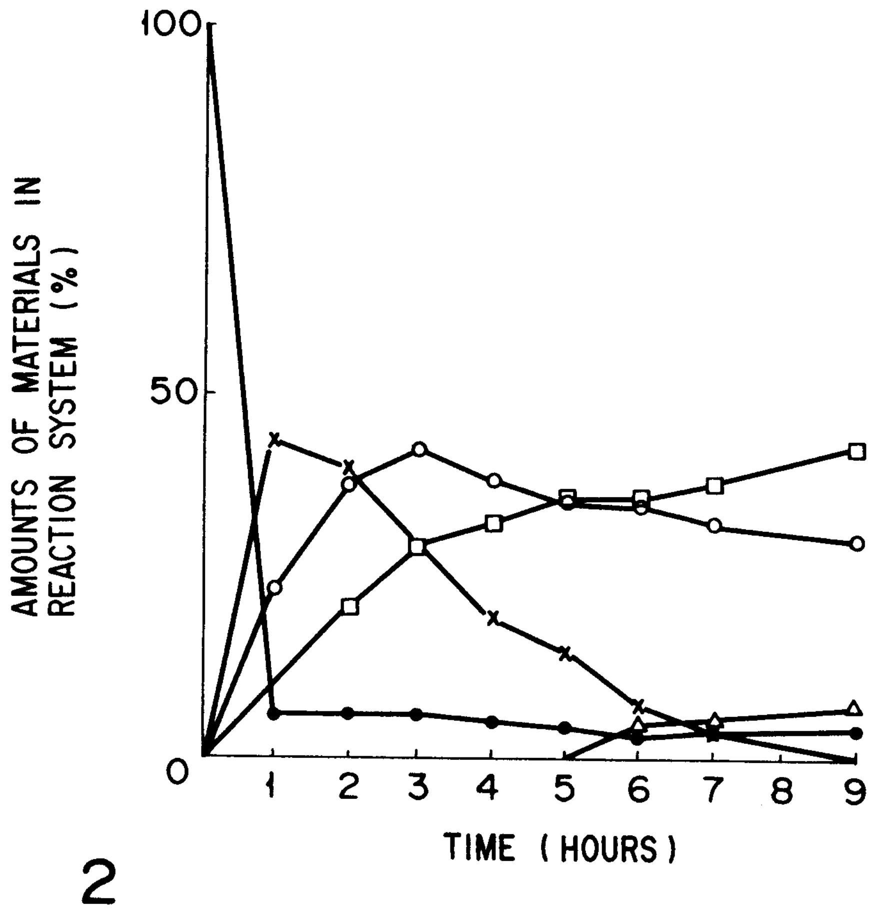
100
50
0
AMOUNTS OF MATERIALS IN REACTION SYSTEM (%)
1 2 3 4 5 6 7 8 9
TIME (HOURS)

FIG. 2

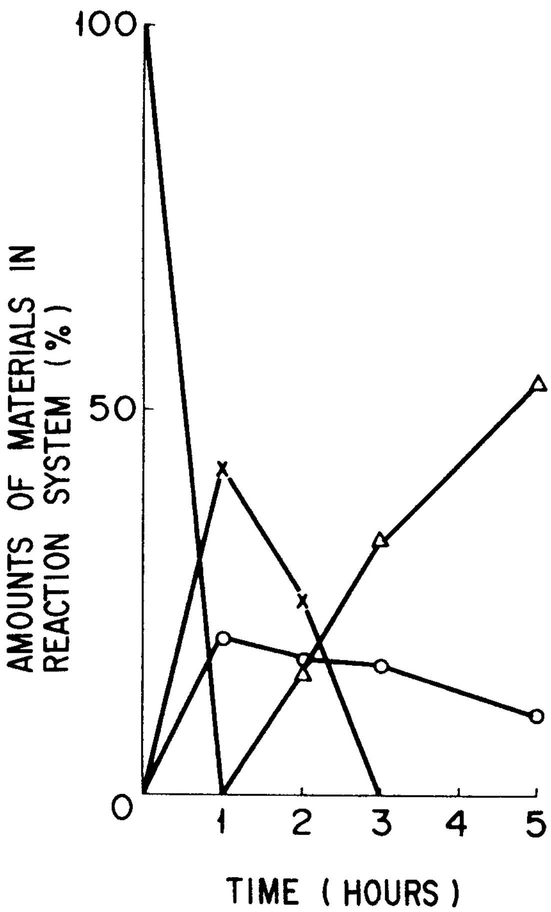
100
50
0
AMOUNTS OF MATERIALS IN REACTION SYSTEM (%)
1 2 3 4 5
TIME ( HOURS )

F I G. 3

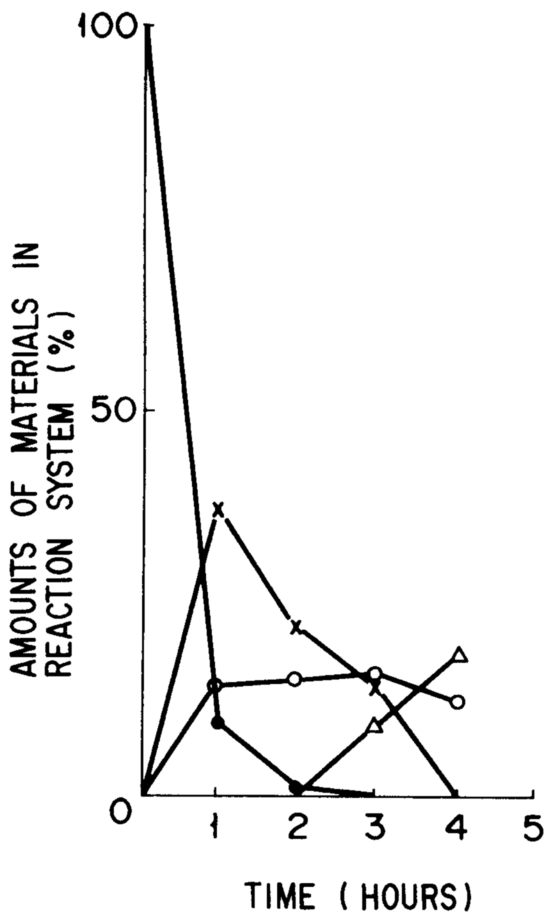
100
50
0
AMOUNTS OF MATERIALS IN REACTION SYSTEM (%)
1 2 3 4 5
TIME ( HOURS)

F I G. 4

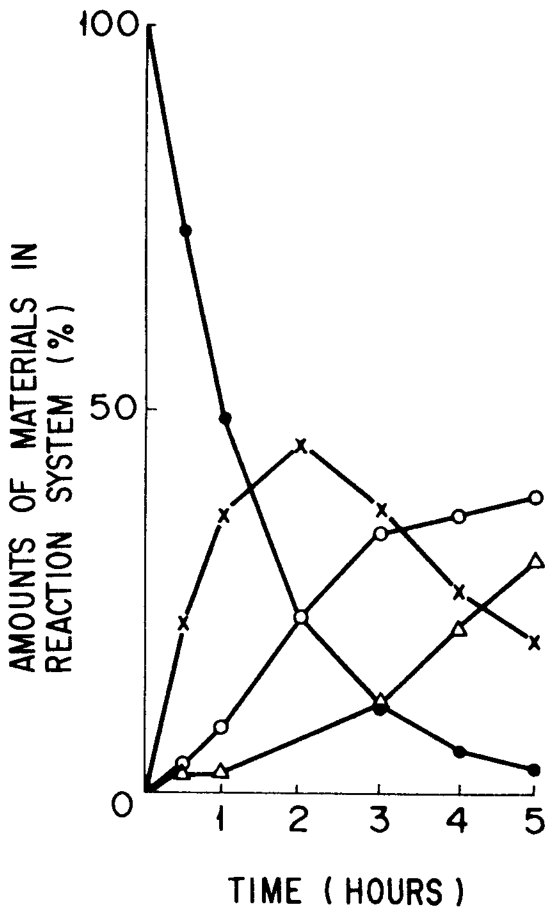
100
50
0
AMOUNTS OF MATERIALS IN
REACTION SYSTEM (%)
1
2
3
4
5
TIME (HOURS)

F I G. 5A

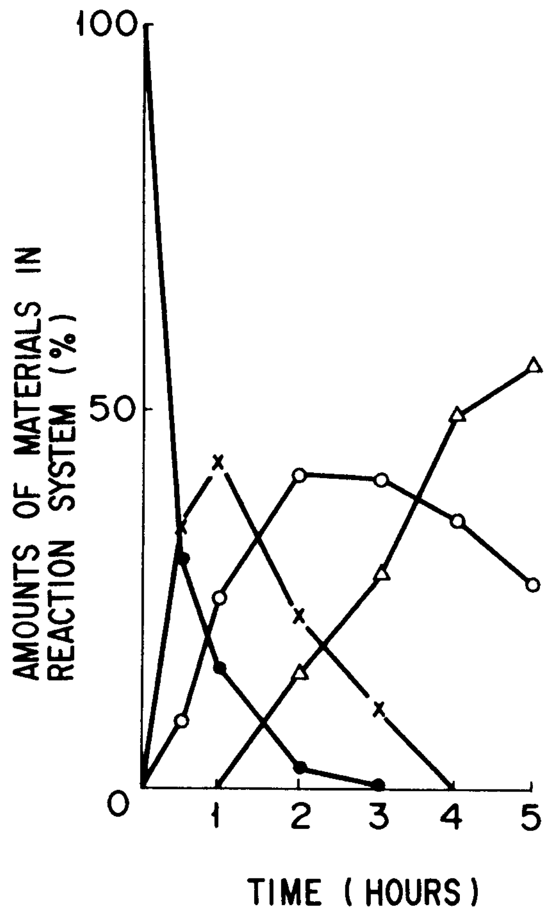
100
50
0
AMOUNTS OF MATERIALS IN
REACTION SYSTEM (%)
1
2
3
4
5
TIME (HOURS)

F I G. 5B

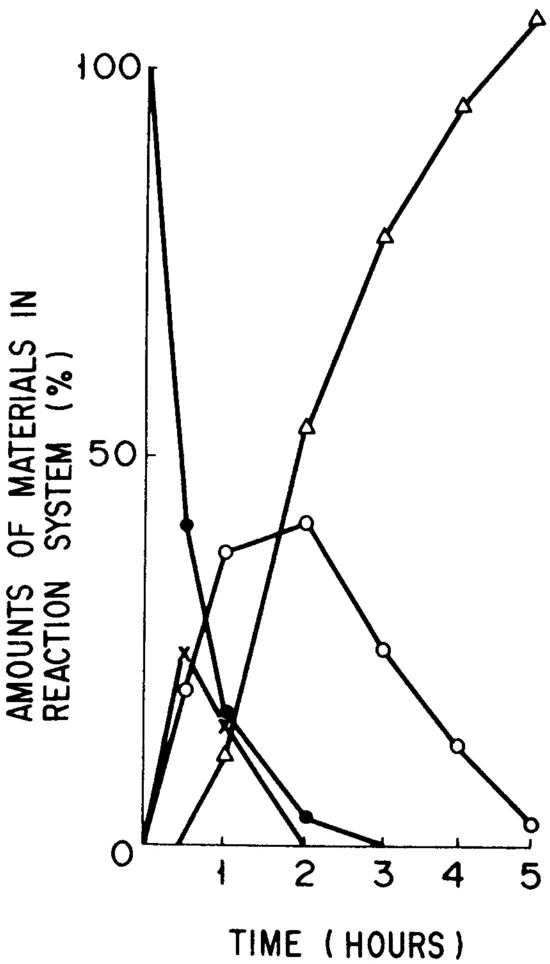
100
50
0
1
2
3
4
5
AMOUNTS OF MATERIALS IN REACTION SYSTEM (%)
TIME (HOURS)

FIG. 5C

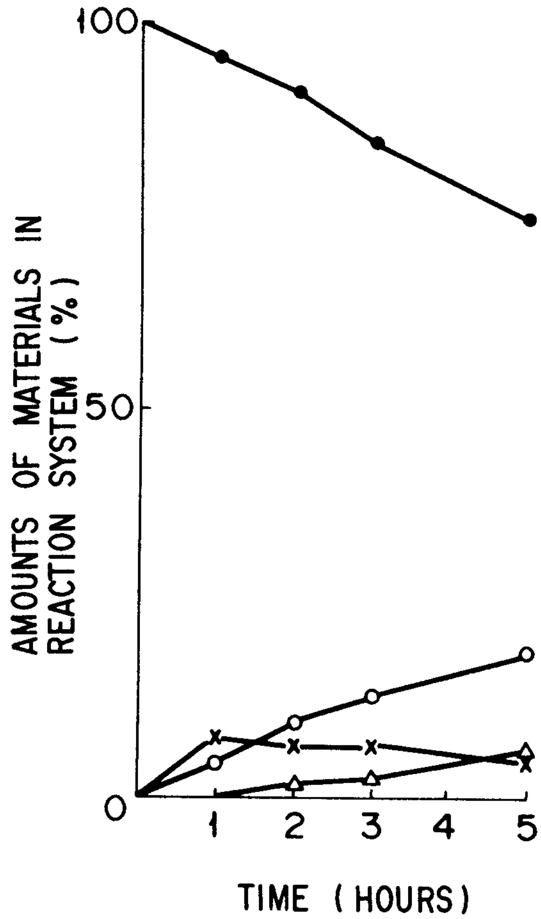
100
50
0
1
2
3
4
5
AMOUNTS OF MATERIALS IN REACTION SYSTEM (%)
TIME (HOURS)

FIG. 6

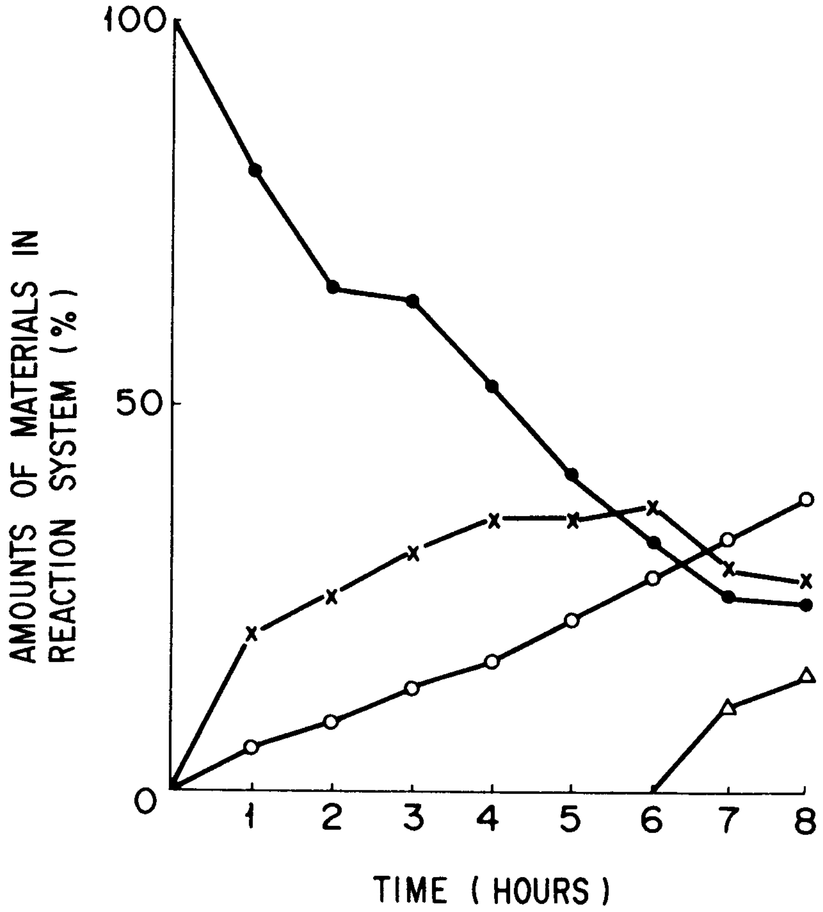
100
50
0
1
2
3
4
5
6
7
8
AMOUNTS OF MATERIALS IN REACTION SYSTEM (%)
TIME (HOURS)

F I G. 7

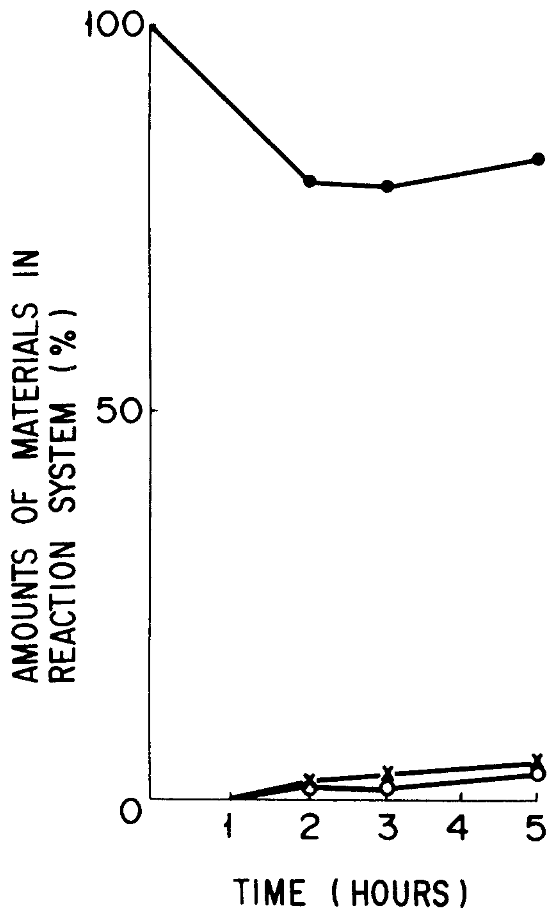
100
50
0
1
2
3
4
5
AMOUNTS OF MATERIALS IN REACTION SYSTEM (%)
TIME (HOURS)

FIG. 8A

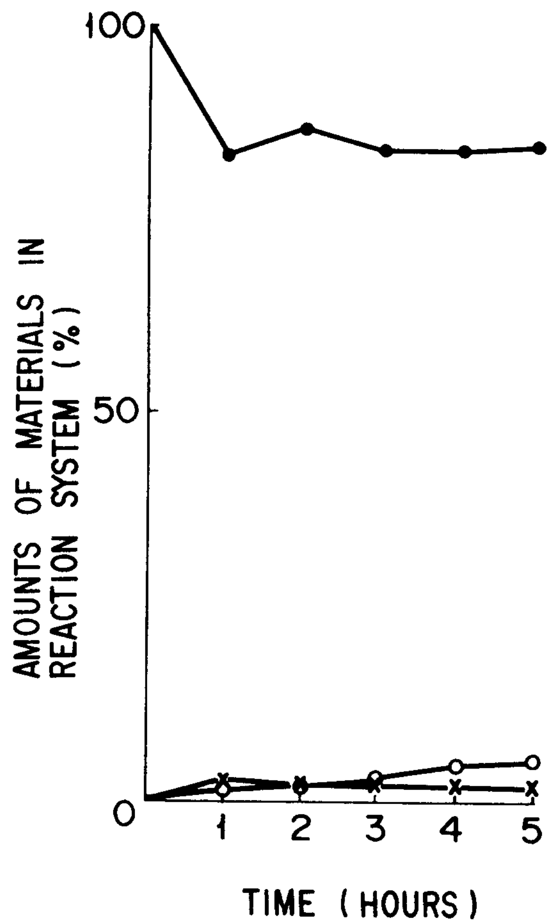
100
50
0
1
2
3
4
5
AMOUNTS OF MATERIALS IN REACTION SYSTEM (%)
TIME (HOURS)

FIG. 8B

# INTERNATIONAL SEARCH REPORT

International Application No PCT/JP91/00857

**I. CLASSIFICATION OF SUBJECT MATTER** (if several classification symbols apply, indicate all) [6]

According to International Patent Classification (IPC) or to both National Classification and IPC

Int. $Cl^5$ C07D405/04, C07D473/34, 473/18, 473/30, 473/06, 473/16, 473/38, 473/32

**II. FIELDS SEARCHED**

Minimum Documentation Searched [7]

| Classification System | Classification Symbols |
|---|---|
| IPC | C07D405/04, 473/34, 473/18, 473/30, 473/06, 473/16, 473/38, 473/32 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, B1, 43-25496 (Kyowa Hakko Kogyo Co., Ltd.), November 4, 1968 (04. 11. 68), (Family: none) | 1-3 |
| A | JP, A, 52-27782 (Kyo Ueda), March 2, 1977 (02. 03. 77), (Family: none) | 1-3 |
| A | JP, A, 52-93779 (Teikoku Kagaku Sangyo K.K.), August 6, 1977 (06. 08. 77), (Family: none) | 1-3 |
| A | JP, A, 53-108982 (Sanyo-Kokusaku Pulp Co., Ltd.), September 22, 1978 (22. 09. 78), (Family: none) | 1-3 |
| A | JP, A, 53-147089 (Ajinomoto Co., Inc.), December 21, 1978 (21. 12. 78), (Family: none) | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 30, 1991 (30. 08. 91) | September 17, 1991 (17. 09. 91) |
| **International Searching Authority** | **Signature of Authorized Officer** |
| Japanese Patent Office | |

International Application No. PCT/JP91/00857

**FURTHER INFORMATION CONTINUED FROM THE SECOND SHEET**

| | | |
|---|---|---|
| A | JP, A, 63-83048 (MECT Corp.), April 13, 1988 (13. 04. 88), (Family: none) | 1-3 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.